# EUROPEAN PATENT SPECIFICATION

(11) **EP 1 064 947 B1**
(45) Date of publication and mention of the grant of the patent: **28.03.2007**
(21) Application number: 99959691.9
(22) Date of filing: 08.12.1999
(51) Int. Cl.: A61K 39/255

(54) **IN OVO VACCINATION OF MAREK'S DISEASE VIRUS TYPE 1**
IN OVO IMPFUNG GEGEN DAS TYP 1 MAREK'S KRANKHEITSVIRUS
VACCINATION IN OVO CONTRE LE VIRUS DE LA MALADIE DE MAREK, TYPE 1

(30) Priority: 14.12.1998 JP 35401798
(43) Date of publication of application: 03.01.2001
(73) Proprietor: Juridical Foundation, The Chemo-Sero-Therapeutic Research Institute, Kumamoto-shi, Kumamoto 860-8568 (JP)
(72) Inventor: YOKOGAWA, Kenji, Kumamoto-shi, Kumamoto 862-8001 (JP); SAKAGUCHI, Masashi, Kumamoto-shi, Kumamoto 860-0864 (JP); TOKUNAGA, Eiji, Kumamoto-shi, Kumamoto 860-0082 (JP)
(74) Representative: Vossius & Partner
(86) International application number: PCT/JP1999/006866
(87) International publication number: WO 2000/035476

(56) References cited:
- EP-A- 0 775 743
- EP-A1- 0 650 733
- EP-A1- 0 774 513
- WO-A-95/35121
- WO-A1-91/04750
- WO-A1-93/14629
- US-A- 4 458 630
- US-A- 5 558 867
- US-A- 5 833 980
- SAKAGUCHI M ET AL: "Protection of chickens with or without maternal antibodies against both Marek's and Newcastle diseases by one-time vaccination with recombinant vaccine of Marek's disease virus type 1" VACCINE, BUTTERWORTH SCIENTIFIC. GUILDFORD, GB, vol. 16, no. 5, 1 March 1998 (1998-03-01), pages 472-479, XP004106960 ISSN: 0264-410X
- MARSHALL D R ET AL: "SELECTION OF MAREK'S DISEASE VIRUS RECOMBINANTS EXPRESSING THE ESCHERICHIA COLI GPT GENE" VIROLOGY, ACADEMIC PRESS,ORLANDO, US, vol. 195, no. 2, 1993, pages 638-648, XP000652334 ISSN: 0042-6822
- SHARMA J.M.: "Delayed replication of arek's disease following in ovo inoculation during late stages of embryonal development" AVIAN DISEASES, vol. 31, 1987, pages 570-576, XP008046375

## Description

### TECHNICAL FIELD OF THE INVENTION

The present invention relates to a prophylaxis for chicken Marek's disease induced by Marek's disease type I virus (hereinafter also referred to as "MDV-1"). More specifically, the present invention relates to a method for immunizing chickens comprising inoculating into a growing chicken egg a composition comprising cell-free attenuated live MDV-1. Furthermore, the present invention provides a method for efficiently immunizing chickens against plural diseases with recombinant MDV-1.

### BACKGROUND OF THE INVENTION

Marek's disease is a infectious disease of chicken caused by MDV-1 and characterized by infiltration or tumorous proliferation of lymphatic cells. It is associated with symptoms such as leg paralysis, formation of lymphoma in various organs of the viscera, weight loss, anemia, diarrhea, etc. In the field, Marek's disease leads to death of the chicken or prompts the poultry inspectorate to order their total or partial destruction and hence results in much economic loss in poultry farmer.

For protection from Marek's disease, attenuated live vaccines have hitherto been used, i.e. MDV-1, MDV-2 or herpes virus of turkey (HVT, MDV-3) alone or in admixture inoculated subcutaneously into newborn chicken. In such a case, a composition of virus-infected cells was usually employed. However, MDV-2 and HVT vaccines are poorly effective against a Marek's disease virus with extremely high pathogenicity, such as the very virulent strain or very virulent (+) reported in recent years. Only a vaccine of MDV-1 can effectively be used (R. L. Witter, AVIAN DISEASE 41: 149-163, 1997).

Nowadays, a number of vaccines have been developed for various chicken infectious diseases. Prophylaxis of diseases by vaccination is a ruling measure for sanitation in the field of poultry farming, irrespective of whether it concerns breeding chicken, laying hen or chicken for meat. On the other hand, labor costs due to scale-up of poultry farming and the crammed schedule of frequent inoculation of vaccine is a burden on the poultry farmer. For obviating this, the development of more effective and efficient vaccination such as reduction in frequency of inoculation by mixing several vaccines (i.e. mixed vaccine) or the improvement of vaccination have been attempted.

As one efficient way of vaccination, Sharma et al. reported vaccination into growing chicken eggs ("in ovo" inoculation) (J. M. Sharma et al., AVIAN DISEASE 26(1): 134-149, 1982). According to Sharma et al., chicken hatched from growing chicken eggs inoculated with HVT-infected cells and chicken inoculated with said HVT after hatching were challenged with Marek's disease virus (hereinafter also referred to as "MDV"). As a result, it was found that the chicken from vaccinated growing eggs exhibited more potent resistance against the challenge, as compared to the chicken vaccinated after hatching, demonstrating that in ovo inoculation can effectively be used for immunization.

Reddy et al. prepared a recombinant HVT (rHVT) wherein a fusion protein (F protein) gene of Newcastle disease virus (hereinafter also referred to as "NDV") and hemagglutinin-neuraminidase (HN) glycoprotein gene of NDV were inserted into the HVT genome (S. K. Reddy et al., Vaccine 14(6): 469-477, 1996). They inoculated cells infected with this virus into growing chicken eggs on day 18. After hatching, the chicken were challenged with both NDV and MDV viruses to thereby demonstrate that the recombinant virus could effectively protect the chicken from the challenge with both viruses. The effect of in ovo vaccination has also been evaluated for an inactivated oil-emulsified vaccine of NDV and avian influenza virus (H. Stone, AVIAN DISEASE 41: 856-863, 1997) and for live Eimeria sporozoite vaccine (Evans et al., WO 96/40233).

Chicken immunization at the stage of a growing egg as described above, called "in ovo vaccination", can be expected to eliminate or reduce labor and cost, and hence an automated approach for inoculation into chicken eggs becomes prevailing.

However, Sharma et al. reported that MDV-1-infected cells were inoculated into growing chicken eggs. The growth of said virus in the chicken embryo tissue was investigated and, no viral growth was observed (J. M. Sharma et al., AVIAN DISEASES 31: 570-576, 1987). They also reported that chicken hatched from said growing eggs were not protected from the challenge with virulent MDV strain. This result suggests that in ovo vaccination against Marek's disease with MDV-1 is not easy. Accordingly, the present situation is that univalent or mixed vaccines of HVT or MDV-2 SB-1 strain have been used instead of MDV-1, the virus causing Marek's disease.

On the other hand, there is a report of an attempt to inoculate MDV-1, which had been regarded as unsuitable for in ovo vaccination, into growing chicken egg (Taniguchi et al., 117th Japan Veterinary Science Association excerpt, p.198, 1994, Tokyo). In this case, when inoculation was made directly into the fetus, the virus efficiently grew and successful immunization was achieved. However, indeed, direct inoculation into the fetus frequently fails. In spite of such circumstances, field application has been promoted because it is thought that even if some individuals failed to be immunized, contact infection occurring about two weeks after MDV-1 inoculation would impart immunization to those individuals that failed to be immunized.

However, if non-immunized chicken are exposed to virulent MDV strain in the field after hatching and prior to infection with said MDV-1 vaccine by contact infection, then said chicken cannot be protected from Marek's disease and hence considerable economic loss will results. Thus, for a thorough protection from Marek's disease, it is most important whether or not immunization is achieved within a week just after hatching. Accordingly, if possible, it is desirable to surely infect the fetus in the growing egg with vaccine virus.

There is also a concern that direct inoculation of a needle into a fetus might possibly damage the fetus badly in some cases.

Under these circumstances, there is a need to develop a more effective MDV-1 vaccine for Marek's disease that is suitable for in ovo vaccination.

### DISCLOSURE OF THE INVENTION

The present inventors thoroughly investigated in order to develop a more effective MDV-1 vaccine for Marek's disease that is suitable for in ovo vaccination. As a result, it was found that a solution containing cell-free virus prepared from a solution of ruptured cells infected with either attenuated MDV-1 or recombinant MDV-1 can be inoculated into growing eggs to thereby exhibit viral growth. It was also found that antibodies to MDV-1 and to other viral antigens incorporated into MDV-1 were induced in serum of chicken that hatched from said virus-inoculated growing egg. In consequence, the present inventors developed the present invention.

An object of the present invention is to provide a method for immunizing chickens, which comprises inoculating into growing egg a composition comprising cell-free attenuated live MDV-1. The present invention also provides a method for efficiently immunizing chickens against plural diseases by using a recombinant MDV-1 in the above method for immunization.

Another object of the present invention is to provide a method for immunizing chickens which comprises inoculating into growing eggs a mixed vaccine comprising the above cell-free MDV-1 plus another vaccine derived from at least one microorganisms selected from the group consisting of viruses other than MDV-1.

The method of the present invention is characterized by a composition comprising cell-free attenuated live MDV-1, a process for preparing the same and in ovo vaccination of said composition.

### BEST MODE FOR CARRYING OUT THE INVENTION

The term "cell-free virus(es)" as used herein means viruses that are have been separated from cells.

For preparing a composition comprising cell-free attenuated live MDV-1, attenuated MDV-1 viruses are infected to host cells and grown therein. The virus-infected cells are then collected by centrifugation at a low speed. For the purpose of the present invention, centrifugation at a low speed is carried out at 1,000 to 3.000 rpm for 3 to 10 minutes using KUBOTA, KN-30F or another centrifuging machine with equivalent turning radius. After addition of a buffer supplemented with an appropriate amount of sugars, the cells are ruptured by sonication or freezing-thawing or by physical means MDV-1 viruses are then extracted from the liquid phase of the ruptured cells and purified. Alternatively, the liquid phase of the ruptured cells may directly be used as it stands without any treatment.

Attenuated MDV-1 includes strains CVI-988, 61-554 (Sakaguchi et al., Japanese Patent Publication No. 6-22757), Md11/75C (R. L. Witter, AVIAN DISEASE 31: 752-765, 1987) and the like, as well as recombinant viruses derived therefrom.

As a host cell for viral infection, any culture cell may be used provided that attenuated MDV-1 can grow therein and it does not produce contaminate viruses. Preferably, avian-derived culture cells are used. Chicken embryo fibroblast cells (CEF cells), duck embryo fibroblast cells, chicken embryo-derived cell strain CHCC-OU2 (Ogura, H. et al., Acta Med Okayama 41(3): 141-143, 1987, and Coussens et al., Japanese Patent Publication No. 9-173059), quail-derived cell strain QT-35 (Spijkers et al., Japanese Patent Publication No. 9-98778) and the like are used, with chicken embryo fibroblast cells (CEF cells) being preferred.

A culture medium for culturing host cells includes a medium commonly used for tissue culture such as M199-earle base (Nissui), Eagle MEM (E-MEM) (Nissui), Dulbecco MEM (D-MEM) (Nissui), SC-UCM102 (Nissui), UP-SFM (GIBCO BRL), EX-CELL302 (Nichirei), EX-CELL293-S (Nichirei), TFBM-01 (Nichirei), ASF104, etc. These culture media are used with supplements of amino acids, salts, anti-fungal or antibacterial agent, animal serum and the like. They may optionally be used as a serum-free medium by not supplementing with serum.

Cells or virus-infected cells are cultured under normal conditions. That is, culture temperature and period may appropriately be adjusted depending on various factors such as types of cells, inoculation amount of viruses and scale and process of culture or a combination thereof. Culture temperatures may range from 35°C to 41°C, preferably from 37°C to 38°C. Culture periods may range from 2 to 7 days, preferably from 3 to 4 days.

The composition of the present invention is inoculated into growing chicken egg. The term "growing chicken egg" as used herein means a fertilized (embryonated) egg in the process of being incubated until 21 days after fertilization, when the chicken is hatched. Vaccination for Marek's disease may be carried out after incubation of 17 to 19 days.

The composition of the present invention may be inoculated into growing chicken eggs, for example, by injecting the viral solution into the air chamber (i.e. at an obtuse-angled site of the egg) of an 18-day old growing egg using an inoculation needle (eg. 24G1·1/4 needle).

In accordance with the composition of the present invention, comprising cell-free attenuated MDV-1 attenuated MDV-1 can efficiently infect in the growing egg and induce immunization for Marek's disease.

The composition of the present invention may also be used for in ovo vaccination as a mixed vaccine in combination with at least one vaccine selected from the group consisting of vaccines to other viruses such as, e.g., avian infectious bronchitis virus, avian infectious bursal disease virus, avian encephalomyelitis virus, egg drop syndrome virus, influenza virus, reovirus, adenovirus, hydropericardium syndrome virus, etc.

The infection-protecting antigen expressed by the recombinant Marek's disease virus used in the present invention includes, in addition to the F protein of Newcastle disease virus exemplified above, the HN protein of Newcastle disease virus, or the core protein, capsid protein or glycoprotein of various viruses (e.g. spike protein or nucleocapsid protein of avian infectious bronchitis virus, VP2 of avian infectious bursal disease virus (IBDV), capsid protein of avian encephalomyelitis virus, capsid protein of egg drop syndrome virus, VP1 + VP2 of avian anemia virus, glycoprotein B of avian infectious laryngotracheitis virus, membrane protein or gag of avian leukemia virus or reticuloendotheliosis virus, glycoprotein of rhinotracheitis of turkeys virus, HA protein of avian pox virus, HA of influenza virus, capsid protein of avian reovirus, capsid protein of avian adenovirus, etc.), cilia, flagella, toxins, hemolysins, membrane proteins such as porin, peptides with antigenicity of O-antigen of bacteria (e.g. Haemophilus paragarinarum, Salmonella choleraesuis, E. coli, Campylobacter, Clostridium, Mycoplasma, enterococcus, etc.), and proteins or glycoproteins from protozoan (e.g. Leucocytozoon caulleryi, Eimeria tenella, E. maxima, E. acervulina, E. brunetti, E. necatrix, avian malaria), and the like.

Such recombinant Marek's disease virus can be prepared using an ordinary process for preparing a recombinant virus well known in the art.

Other than ND, antigens that were actually incorporated into a recombinant MDV-1 and reported to be immunogenic in chicken includes IBDV-VP2 antigen, as reported by Tsukamoto et al., Virology 257: 352-362, 1999. A number of recombinant viruses incorporating the marker gene, lacZ, were also reported (Sakaguchi et al., Virology 195: 140-148, 1993; Percells et al., J. Virology 68: 8239-8253, 1994; Schat et al., J. gen. Virology 70: 841-849, 1998)

The present invention is explained in more detail by means of the following Examples.

### EXAMPLE

### Example 1: In ovo vaccination of MDV-1

### (1) Preparation of materials for inoculation of growing chicken egg

About 1 x 10⁸ CEF cells and about 1 x 10⁶ PFU of MDV-1 Rispens strain were suspended in E-MEM medium (40 ml) supplemented with 5% fetal bovine serum (FBS). The suspension was placed in a 175 cm² culture flask and incubated at 37°C for 4 days. When 80% or more cytopathic effect (CPE) was observed, virus-infected cells were collected in the usual manner using 0.1% EDTA - 0.125% trypsin (DIFCO). The collected cells were suspended in 40 ml of E-MEM medium. From this suspension, materials for inoculation into growing chicken egg were prepared by the following two processes:
(a) A solution of virus-infected cells:
   The above suspension (1 ml) was serially diluted 10-fold with E-MEM to prepare a solution of virus-infected cells (as a reference control, not part of the invention).
(b) A solution of cell-free viruses:
   The remaining suspension (39 ml) was centrifuged at a low speed at 1,500 rpm for 5 minutes. After removing the supernatant by suction, the cells were suspended by adding 2 ml of SPGA-SBT solution (prepared in accordance with B. W. Calnek et al., Appl. Microbiol. (20): 723-726, 1970; B. R. Cho, Avian Dis. 22(1): 170-176, 1977; 0.218 M sucrose, 0.0038 M potassium dihydrogenphosphate, 0.0072 M dipotassium hydrogenphosphate, 0.0049 M sodium glutamate, 1% bovine serum albumin, 10% sorbitol). The suspension was sonicated with TOMY SEICO Co., LTD, Handy sonic UR-20P, Power cont. 4 for 1 minute and then centrifuged at a low speed at 2,500 rpm for 5 minutes. The supernatant was serially diluted 10-fold to prepare a solution of cell-free viruses.
   Viruses in the inoculation materials were quantified as follows: CEF cells previously cultured were collected with EDTA-trypsin and centrifuged at 1,500 rpm for 5 minutes. The obtained cellular sediment was again suspended in 5%-FBS at a concentration of 6 x 10⁵ cells/ml (hereinafter referred to as "CEF2nd").
   The CEF2nd cells (9 x 10⁶ cells/15 ml in a 10 cm Petri dish) were cultured for 4 hours and inoculated with 1 ml of a vaccine solution of each dilution by tilting every 20 minutes for 1 hour. The cells were then added with E-MEM medium (5 ml) and incubated in a CO₂ incubator at 37°C overnight. The next day, E-MEM supplemented with 2% methyl cellulose (Sigma) and 1% FCS was overlaid, and the cells were incubated in the CO₂ incubator at 37°C for 10 days. The number of plaques that appeared was counted.

### (2) Inoculation into growing chicken egg

Each group consisted of six growing chicken eggs 18 days old (SPF manufactured by Juridical Foundation The Chemo-Sero-Therapeutic Research Institute). An aliquot of 0.1 ml of each of the solutions of virus-infected cells serially diluted 10-fold, respectively an aliquot of 0.1 ml of each of the solutions of cell-free viruses were inoculated into the eggs by injecting with a 24G1·1/4 inoculating needle (Nipro) into an air chamber (an obtuse angled site) of the egg with about 2.5 cm depth. The eggs were incubated at 37°C for 3 days. A week after hatching, 1 ml of blood was drawn from the heart with a heparinized 5 ml disposable syringe (Nipro) and a 21G 1 inch needle (Nipro). A fraction of mononuclear cells was separated from said blood using Ficoll Paque Plus (Pharmacia) in accordance with the manufacture's instruction. The whole of this fraction was inoculated into CEF2nd cells (9 x 10⁶ cells/15 ml, in Petri dish of 10 cm diameter) previously cultured for 4 hours. Ten days later, plaques of Marek's disease viruses appeared and were counted. Recovery rates of viruses in the group inoculated with the solution of virus-infected cells and in the group inoculated with the solution of cell-free viruses are shown in Tables 1 and 2, respectively. Blood was drawn from 5 chicken in each of the groups to examine viral recovery.

**Table 1**

| Inoculated amount per chicken (PFU) | Rate of viral infection (Positive No./Total No.) |
|---|---|
| 1280 | 4/5 |
| 128 | 2/5 |
| 12.8 | 0/5 |
| 1.28 | 0/5 |

**Table 2**

| Inoculated amount per chicken (PFU) | Rate of viral infection (Positive No./Total No.) |
|---|---|
| 540 | 5/5 |
| 54 | 5/5 |
| 5.4 | 5/5 |
| 0.54 | NT |

As apparent from Tables 1 and 2, viruses were recovered from merely less than the half of the individuals among chicken from eggs inoculated with 128 PFU in the group inoculated with the solution of virus-infected cells. In contrast, in the group inoculated with the solution of cell-free viruses, viruses were recovered from all the five chicken, even in the group inoculated with as low as 5.4 PFU. This demonstrates that MDV-1 viruses indeed propagate in case of the solution of cell-free viruses and hence it can be effectively used.

### Example 2: In ovo vaccination of recombinant MDV-1

### (1) Preparation of materials for inoculation of growing chicken egg

About 1 x 10⁸ CEF cells and about 1 x 10⁶ PFU of recombinant virus rMDV1 US10P(F) strain wherein a gene for Newcastle disease virus F (NDV-F) protein was incorporated (Sonoda et al., Current research on Marek's disease, p.408, 1996) were suspended in E-MEM medium (40 ml) supplemented with 5% fetal bovine serum (FBS). The suspension was placed in a 175 cm² culture flask and incubated at 37°C for 4 days. When 80% or more cytopathic effect (CPE) was observed, virus-infected cells were collected in the usual manner using 0.1% EDTA - 0.125% trypsin (DIFCO). The collected cells were suspended in 10 ml of E-MEM medium. From this suspension, materials for inoculation into growing chicken egg were prepared by the following processes:
Preparation of a solution of cell-free viruses:
   As described in Example 1 (1), the suspension (40 ml) was centrifuged at a low speed at 1,500 rpm for 5 minutes. After removing supernatant by suction, the cells were suspended by adding 2 ml of SPGA-SBT solution. The suspension was sonicated for 1 minute and then centrifuged at a low speed at 2,500 rpm for 5 minutes. The obtained supernatant was serially diluted 10-fold to prepare a solution of cell-free viruses.

As described in Example 1(1), viruses in the inoculation materials were counted as follows: i.e. the CEF2nd cells (9 x 10⁶ cells/15 ml in a Petri dish of 10 cm diameter) were prepared as described in Example 1(1) and previously cultured for 4 hours. The cells were inoculated with 1 ml of each dilution of the vaccine solution by tilting every 20 minutes for 1 hour. The cells were then added with E-MEM medium (5 ml) and incubated in a CO₂ incubator at 37°C overnight. The next day, E-MEM supplemented with 2% methyl cellulose (Sigma) and 1% FCS was overlaid, the cells were incubated in the CO₂ incubator at 37°C for 10 days and the number of plaques that appeared was counted.

### (2) Inoculation into growing chicken eggs

Each group consisted of five growing chicken eggs 18 days old (SPF manufactured by Juridical Foundation The Chemo-Sero-Therapeutic Research Institute).

A 0.1 ml aliquot of each of the solutions of cell-free viruses was inoculated into the eggs by injecting with a 24G1·1/4 inoculating needle (Nipro) into an air chamber (an obtuse angled site) of the egg with about 2.5 cm depth. The eggs were incubated at 37°C for 3 days. A week after hatching, 1 ml of blood was drawn from the heart with a heparinized 5 ml disposable syringe (Nipro) and 21G 1 inch needle (Nipro). A fraction of mononuclear cells was separated from said blood using Ficoll Paque Plus (Pharmacia) in accordance with the manufacture's instruction. The whole of this fraction was inoculated into CEF2nd cells (9 x 10⁶ cells/15 ml, in Petri dish of 10 cm diameter) previously cultured for 4 hours. Ten days later, plaques of Marek's disease virus appeared and were counted.

**Table 3**

| Inoculated amount per chicken (PFU) | Rate of viral infection (Positive No./Total No.) | Average Viremia¹⁾ |
|---|---|---|
| 320 | 5/5 | 3.27 |
| 32 | 5/5 | 1.82 |
| 3.2 | 3/5 | 1.61 |
| 0.32 | 0/5 | NT |

| | | |
|---|---|---|
| 1) Viral amount per 1 x 10⁶ mononuclear cells | | |

As apparent from Table 3, viruses were recovered from all the five chicken, even in the group inoculated with as low as 32 PFU. Thus, effectiveness of the solution of cell-free viruses was confirmed in case of recombinant MDV-1 virus.

### (3) Antibody test

Growing chicken eggs were inoculated as described above. Each group consisted of six growing chicken eggs 18 days old (SPF manufactured by Juridical Foundation The Chemo-Sero-Therapeutic Research Institute). When chicken hatched from the eggs became 8 weeks old, blood was drawn and the level of antibody to NDV-F protein was determined.

The antibody was detected by ELISA with NDV-F expressing cells as an antigen. Details of this procedure are described in Sakaguchi M. et al., Vaccine, 1996 June, 14(8): 747-52. The levels of NDV-F antibody positive individuals in the group inoculated with the solution of virus-infected cells and in the group inoculated with the solution of cell-free viruses are shown in Tables 4 and 5, respectively.

**Table 4**

| Inoculated amount per chicken (PFU) | Rate of NDV-F Ab positive individuals (Positive No./Total No.) |
|---|---|
| 84 | 2/6 |
| 8.4 | 0/6 |

**Table 5**

| Inoculated amount per chicken (PFU) | Rate of NDV-F Ab positive individuals (Positive No./Total No.) |
|---|---|
| 70 | 5/5 |
| 7 | 4/6 |
| 0.7 | 2/6 |
| No inoculation | 0/6 |

As shown in Table 5, all the chickens were positive for the antibody in the group of chickens from the eggs inoculated with 70 PFU cell-free viruses. In the group of chickens from the eggs inoculated with 7 PFU cell-free viruses, four among six chickens were positive. In contrast, in the group of chickens inoculated with the solution of virus-infected cells, only two among six chickens were found to be positive when 84 PFU was inoculated. Thus, the group inoculated with cell-free virus showed a higher positive conversion even with a 10-fold lower amount of viruses than in the group treated with virus-infected, demonstrating. The usefulness of the cell-free viruses for immunization of growing eggs in view of antibody response.

### INDUSTRIAL APPLICABILITY

In accordance with the present invention, a cell-Free MDV-1 live vaccine for prophylaxis of chicken Marek's disease and a method for immunization by in ovo inoculation using said live vaccine, are provided. The method of the present invention allows for protection from Marek's disease virus infection more efficiently and effectively as compared to the conventional methods. Furthermore, the method of the present invention also allows for immunization for foreign gene products simultaneously expressed by MDV-1 in a laborsaving manner.

## Claims

1. Use of a composition comprising cell-free attenuated live viruses of Marek's disease type 1 for the preparation of a vaccine for the immunization of chicken, wherein said vaccine is to be inoculated into a growing egg.

2. Use of a mixed vaccine comprising cell-free attenuated viruses of Marek's disease type 1plus another vaccine from at least one microorganism selected from the group consisting of viruses other than MDV-1 for the preparation of a vaccine for the immunization of chicken, wherein said vaccine is to be inoculated into a growing egg.

3. The use of claim 2 wherein said microorganisms are selected from the group consisting of avian infectious bronchitis virus, avian infectious bursal disease virus, avian encephalomyelitis virus, egg drop syndrome virus, influenza virus, reovirus, adenovirus and hydropericardium syndrome virus.

4. The use of claim 2 or 3 wherein said microorganisms are not inactivated

5. The use of claims any one of 1to 4 wherein said attenuated viruses of Marek's disease type 1 are a recombinant virus of Marek's disease type 1.

6. The use of claim 5 wherein genes incorporated into said recombinant virus of Marek's disease type 1 code for antigens from viruses other than virus of Marek's disease type 1, bacteria or protozoan.

7. The use of claim 6 wherein said antigens are selected from the group consisting of F protein and HN protein of Newcastle disease virus, spike protein or core protein of avian infectious bronchitis virus, VP2 of avian infectious bursal disease virus (IBDV), capsid protein of avian encephalomyelitis virus, capsid protein of egg drop syndrome virus, VP1 + VP2 of avian anemia virus, glycoprotein B of avian infectious laryngotracheitis virus, membrane protein or gag of avian leukemia virus or reticuloendotheliosis virus, glycoprotein of rhinotracheitis of turkeys virus, HA protein of avian pox virus, HA of influenza virus, capsid protein of avian reovirus, capsid protein of avian adenovirus, cilia, flagella, toxins, hemolysins, membrane proteins, peptides with antigenicity of O-antigen and proteins or glycoproteins from protozoan.

## Patentansprüche

1. Verwendung einer Zusammensetzung umfassend zellfreie attenuierte lebende Marek's Disease Typ 1-Viren für die Herstellung eines Impfstoffes zur Immunisierung von Hühnern, wobei der Impfstoff in ein wachsendes Ei geimpft werden soll.

2. Verwendung eines Mischimpfstoffs umfassend zellfreie attenuierte Marek's Disease Typ 1-Viren plus einen anderen Impfstoff von mindestens einem Mikroorganismus, ausgewählt aus der Gruppe bestehend aus anderen Viren als MD V-1, für die Herstellung eines Impfstoffs zur Immunisierung von Hühnern, wobei der Impfstoff in ein wachsendes Ei geimpft werden soll.

3. Verwendung nach Anspruch 2, wobei die Mikroorganismen ausgewählt sind aus der Gruppe bestehend aus aviärem infektiöse Bronchitis-Virus, aviärem infektiöse Bursakrankheit-Virus, aviärem Encephalomyelitis-Virus, Egg-Drop-Syndrom-Virus, Influenzavirus, Reovirus, Adenovirus und Hydropericardium-Syndrom-Virus.

4. Verwendung nach Anspruch 2 oder 3, wobei die Mikroorganismen nicht inaktiviert sind.

5. Verwendung nach einem der Ansprüche 1 bis 4, wobei die attenuierten Marek's Disease Typ 1-Viren ein rekombinantes Marek's Disease Typ 1-Virus sind.

6. Verwendung nach Anspruch 5, wobei Gene, welche in das rekombinante Marek's Disease Typ 1-Virus eingebaut sind, Antigene von anderen Viren als dem Marek's Disease Typ 1-Virus, von Bakterien oder von Protozoen codieren.

7. Verwendung nach Anspruch 6, wobei die Antigene ausgewählt sind aus der Gruppe bestehend aus dem F-Protein und HN-Protein des Newcastle Disease-Virus, dem Spikeprotein oder Kernprotein des aviären infektiöse Bronchitis-Virus, VP2 des aviären infektiöse Bursakrankheit-Virus (IBDV), dem Capsidprotein des aviären Encephalomyelitis-Virus, dem Capsidprotein des Egg-Drop-Syndrom-Virus, VP1 und VP2 des Hühner-Anämie-Virus, dem Glycoprotein B des aviären infektiöse Laryngotracheitis-Virus, dem Membranprotein oder gag des Geflügel-Leukämie-Virus oder -Reticuloendotheliosis-Virus, dem Glycoprotein des Truthahn-Rhinotracheitis-Virus, dem HA-Protein des Geflügel-Pockenvirus, HA des Influenzavirus, dem Capsidprotein des Geflügel-Reovirus, dem Capsidprotein des Geflügel-Adenovirus, Cilien, Flagellen, Toxinen, Hämolysinen, Membranproteinen, Peptiden mit der Antigenität des O-Antigens und Proteinen oder Glycoproteinen aus Protozoen.

## Revendications

1. Utilisation d'une composition comprenant des virus vivants atténués acellulaires de la maladie de Marek de type 1 pour la préparation d'un vaccin pour l'immunisation du poulet, dans laquelle ledit vaccin doit être inoculé dans un oeuf en croissance.

2. Utilisation d'un vaccin associé comprenant des virus atténués acellulaires de la maladie de Marek de type 1 plus un autre vaccin à partir d'au moins un microorganisme sélectionné dans le groupe constitué de virus autres que MDV-1 pour la préparation d'un vaccin pour l'immunisation du poulet, dans laquelle ledit vaccin doit être inoculé dans un oeuf en croissance.

3. Utilisation selon la revendication 2 dans laquelle lesdits microorganismes sont sélectionnés dans le groupe constitué du virus de la bronchite infectieuse aviaire, du virus de la bursite infectieuse aviaire, du virus encéphalomyélite aviaire, du virus du syndrome de chute de ponte, du virus de la grippe, du réovirus, de l'adénovirus et du virus du syndrome de l'hydropéricarde.

4. Utilisation selon la revendication 2 ou 3 dans laquelle lesdits microorganismes ne sont pas inactivés.

5. Utilisation selon l'une quelconque des revendications 1 à 4 dans laquelle lesdits virus atténués de la maladie de Marek de type 1 sont un virus recombinant de la maladie de Marek de type 1.

6. Utilisation selon la revendication 5 dans laquelle les gènes incorporés dans ledit virus recombinant de la maladie de Marek de type 1 codent des antigènes de virus autres que le virus de la maladie de Marek de type 1, de bactéries ou d'un protozoaire.

7. Utilisation selon la revendication 6 dans laquelle lesdits antigènes sont sélectionnés dans le groupe constitué de la protéine F et de la protéine HN du virus de la maladie de Newcastle, de la protéine de spicule ou de la protéine du core du virus de la bronchite infectieuse aviaire, du VP2 du virus de la bursite infectieuse aviaire (IBDV), de la protéine de capside du virus de l'encéphalomyélite aviaire, de la protéine de capside du virus du syndrome de chute de ponte, du VP1+VP2 du virus de l'anémie aviaire, de la glycoprotéine B du virus laryngotrachéite infectieuse aviaire, de la protéine membranaire ou gène gag du virus de la leucémie aviaire ou du virus de la réticulo-endothéliose, de la glycoprotéine du virus rhinotrachéite de la dinde, de la protéine HA du virus de la variole aviaire, de la protéine HA du virus de la grippe, de la protéine de capside du réovirus aviaire, de la protéine de capside de l'adénovirus aviaire, de cils vibratiles, de flagelles, de toxines, d'hémolysines, de protéines membranaires, de peptides avec antigénicité de l'antigène O et de protéines ou glycoprotéines de protozoaire.
